# EUROPEAN PATENT APPLICATION

(11) **EP 2 436 698 A1**
(43) Date of publication of application: **04.04.2012**
(21) Application number: 10186233.2
(22) Date of filing: 01.10.2010
(51) Int. Cl.: C07K 19/00, C12N 9/24, C12P 7/02

(54) **Cellulolytic Clostridium acetobutylicum**

(71) Applicant: Institut National des Sciences Appliquées (INSA), 31077 Toulouse Cedex 4 (FR)
(72) Inventor: Soucaille, Philippe, 31450 Deyme (FR); Croux, Christian, 31320 Auzeville Tolosane (FR); Meynial, Isabelle, 31450 Fourquevaux (FR)
(74) Representative: Tetaz, Franck Claude Edouard

(57) **Abstract**

The present invention relates to a new strain of *Clostridium acetobutylicum* modified to be able to grow on cellulose wherein the wild type *cel48A* gene encoding an inactive Ce148A cellulase is replaced with a hybrid gene coding for an active family 48 glycoside hydrolase catalytic domain and a dockering domain that can bind the cohesive domain of the CipA protein and its use for the production of bulk chemicals by conversion of cellulose as source of carbon, such as ethanol, butanol, glycerol, 1,2-propanediol, 1,3-propanediol, acetone, isopropanol, hydrogen, acetic acid and lactic acid and the like.

## Description

### DOMAIN OF THE INVENTION

The present invention relates to a new strain of *Clostridium acetobutylicum* modified to be able to grow on cellulose and its use for the production of bulk chemicals by conversion of cellulose as source of carbon, such as ethanol, butanol, glycerol, 1,2-propanediol, 1,3-propanediol, acetone, isopropanol, hydrogen, acetic acid and lactic acid and the like.

### BACKGROUND OF THE INVENTION

*Clostridium acetobutylicum* is known for nearly 100 years to produce solvents such as butanol, as well as other bulk chemicals. However, if *Clostridium acetobutylicum* can utilize all the sugars resulting from cellulose hydrolysis (Compere, A.L., and W.L. Griffith. 1979) and hemicellulose (Dunning, J.W., and E.C. Lathrop. 1945; Nakhamanovich, B.M., and N.A. Sticheblykina. 1959) and degrades polymers such as starch or xylan, it is not able to hydrolyze and grow on crystalline cellulose (Lee, S.F& al. 1985).

Cellulosome, a macromolecular complex for cellulose degradation (Bayer E.A., Y. Shoham, and R. Lamed. 2000), has been genetically and biochemically characterized in four *Clostridium* species including C. *cellulolyticum* (Gal, L & al. 1997), C. *cellulovorans* (Doi, R.H. & al. 1994), *C. josui* (Kakiuchi, M. & al. 1998.) and C. *thermocellum* (Lamed, R., and E.A. Bayer. 1988), but not in C. *acetobutylicum.*

Surprisingly, the genome sequencing of C. *acetobutylicum* ATCC 824 (Nolling et al.) has revealed the presence of a large cellulosomal gene cluster. Sequence analysis revealed that this cluster contains the genes for the CipA scaffolding protein, the Ce148A cellulase, several cellulases of family 5 and 9, the Man5G mannanase, and an hydrophobic protein, OrfXp. Genetic organization of this large cluster is very similar to those of the mesophilic C. *cellulolyticum* and C. *cellulovorans* (Bélaïch, J.P. & al. 1997; Tamaru, Y. & al. 2000).

As C. *acetobutylicum* is unable to grow on cellulosic substrates, the existence of a cellulosomal gene cluster in the genome raises questions about its expression, function and evolution. In an earlier work, we have established that C. *acetobutylicum* can produce a cellulosome with an apparent molecular weight of about 665 kDa (Sabathé, F. & al. 2002). Biochemical and immunochemical analyses of the cellulosomal components revealed the existence of at least four subunits including the CipA scaffolding protein and three major cellulases, Ce148A, Ce19X, and Ce19C cellulases.

The reason why the cellulosome system from C. *acetobutylicum* is inactive remains unknown whilst there is a need for cellulolytic C. *acetobutylicum* strains for the production of bulk chemical from biomass.

The present invention is based on recent finding by the inventors that i) Ce19C and Ce19X, two of the three major cellulases of the cellulosome of C. *acetobutylicum* are both active on crystalline cellulose and that they can form a complex with the CipA scaffolding protein, and Ce148A, the major cellulase of the cellulosome is inactive on cellulose explaining the lack of activity of the C. *acetobutylicum* cellulosome on cellulose.

### BRIEF DESCRIPTION OF THE INVENTION

The present invention concerns a genetically modified *Clostridium acetobutylicum* able to grow on cellulose, wherein it comprises a hybrid gene coding for an active family 48 glycoside hydrolase with a dockerin domain that can bind to the cohesin domains of the *Clostridium acetobutylicum* CipA scaffolding protein.

The invention also concerns a method for the preparation of a genetically modified *Clostridium acetobutylicum* able to grow on cellulose, comprising replacing the wild type gene *cel48A* encoding an inactive Ce148A cellulase with an hybrid gene coding for an active family 48 glycoside hydrolase and a dockerin domain that could bind to the cohesin domains of the *Clostridium acetobutylicum* CipA scaffolding protein , said gene coding for an active family 48 cellulase being functional in *Clostridium acetobutylicum.*

The invention also concerns a method for preparing such genetically modified *Clostridium acetobutylicum* comprising a further step of adaptation with the steps of
- repeated subcultures on cellulose of the modified *Clostridium acetobutylicum* and
- selection of the modified strains of *Clostridium acetobutylicum* having an improved ability to grow on cellulose.

The modified *Clostridium acetobutylicum* is also modified for an improved production of a targeted bulk chemical, by attenuating and/or deleting and/or replacing genes to favor a metabolic pathway for the production of the targeted bulk chemical.

The invention also concerns a method for the production of a targeted bulk chemical from cellulose, comprising culturing a genetically modified *Clostridium acetobutylicum* of the invention on a culture medium comprising cellulose as main source of carbon, and recovering the targeted bulk chemical from the culture medium.

Bulk chemicals chemical susceptible to be produced by culturing *Clostridium acetobutylicum* are known in the art and are preferably selected among the group consisting of ethanol, butanol, glycerol, 1,2-propanediol, 1,3-propanediol, acetone, isopropanol, hydrogen, acetic and lactic acids, and mixtures thereof.

### DETAILED DESCRIPTION OF THE INVENTION

In the present application, terms are employed with their usual meaning, except when precised otherwise.

"Cellulose" means a polysaccharide consisting of a linear chain of several hundred to over ten thousand β(1 → 4) linked D-glucose units. Preferred forms of cellulose for the present invention are selected among crystalline cellulose, phosphoric acid swollen cellulose and lignocellulosic biomass pretreated by steam explosion or ammonia fiber expansion, or organosolve or sulfite

*"Clostridium acetobutylicum"* is known in the art and means strain producing acetone butanol and ethanol and possessing a megaplasmid carrying the solvent forming genes. Preferred strains of *Clostridium acetobutylicum* used in the present invention are strains known to be used and modified for the production of solvents and other bulk chemicals, such as *Clostridium acetobutylicum* ATCC 824, *Clostridium acetobutylicum* DSM 1731 and *Clostridium acetobutylicum* ATCC 4259.

"bulk chemical(s)" means large volume chemicals. Preferred bulk chemical susceptible to be produced with the strain of the invention are selected among the group consisting of ethanol, butanol, glycerol, 1,2-propanediol, 1,3-propanediol, acetone, isopropanol, acetic acid and lactic acids, and mixtures thereof.

Transformation of cellulose for the production of the following bulk chemical have been disclosed in the art, including the production of ethanol (Lynd L.R. & al, 2005), butanol (Weber C. & al, 2010) acetone (Weber C. & al, 2010), acetic acid and lactic acid (Maki M; & al , 2009).

The hybrid gene of the invention can indeed be introduced in the *Clostridium acetobutylicum* by usual methods. It can be introduced in the strain on aproduction plasmid, wherein the coding sequence is under control of regulatory elements allowing expression and translation of the gene in *Clostridium acetobutylicum.* It can also be integrated in the genome of the strain.

Preferably, the hybrid gene of the invention replaces the wild type *cel48A* gene of *Clostridium acetobutylicum* encoding an inactive Ce148A cellulose.

"Gene replacement" means replacing in the genome of *Clostridium acetobutylicum* a gene sequence or part of the gene sequence with another gene sequence. The full gene sequence, including regulatory elements or part of the regulatory elements may be replaced with an heterologous gene sequence comprising regulatory elements functional in *Clostridium acetobutylicum.*

In another embodiment, only the sequence coding for the wild type inactive Ce148A cellulase of the wild type *cel48A* gene is replaced with the coding sequence of a hybrid gene coding for an active family 48 glycoside hydrolase and a dockerin domain that could bind to the cohesin domains of the *Clostridium acetobutylicum* CipA scaffolding protein.

In another embodiment, the sequence coding for the dockerin domain of the *cel48A* gene is conserved and only the sequence coding for the inactive family 48 glycoside hydrolase catalytic domain is replaced with a corresponding sequence, comprising a sequence coding for an active catalytic domain of an active family 48 glycoside hydrolase .

Active family 48 glycoside hydrolase with an active catalytic domain are know in the art and preferably selected among the group consisting of the proteins and genes listed on Table 1. In a preferred embodiment, the active family 48 glycoside hydrolase susceptible to replace the wild type inactive Ce148A cellulase, comprise an active catalytic domain as disclosed in SEQ ID NO 1. Preferably, only the sequence coding for the inactive catalytic domain in the wild type *cel48A* gene will be replaced by a corresponding sequence coding for an active cellulase domain.

Dockerin domains that can bind to the cohesin domains of the *Clostridium acetobutylicum* CipA scaffolding protein are known in the art and preferably selected among the group consisting of the proteins and genes listed on Table 2. In a preferred embodiment, the dockerin domain susceptible to be associated to an active catalytic domain is disclosed in SEQ ID NO 2.

The hybrid protein of the invention advantageously comprises the catalytic domain of an active family 48 glycoside hydrolase and the dockerin domain that bind to the cohesin domains of the *Clostridium acetobutylicum* CipA scaffolding protein, as defined above.

In a preferred embodiment, the hybrid protein comprises the amino acid sequence depicted in SEQ ID NO 3, comprising the dockerin domain of SEQ ID NO 2 and the active catalytic domain of SEQ ID NO 1.
SEQ ID NO 3 : hybrid protein Ce148SAFA consisting in:
- Signal peptide of Ce148A from C. *acetobutylicum*
- Catalytic domain of CEL48F from C. *cellulolyticum*
- Dockerin domain of Ce148A from C. *acetobutylicum* mlkisknfkkimavaltstvifgslsglltnkvaaatttankvyqdrfesmyskikdpangyfseqgipyhsietlmveapdyg hvttseamsyymwleamhgrfsgdftgfdkswsvteqyliptekdqpntsmsrydankpatyapefqdpskypspldtsq pvgrdpinsqltsaygtsmlygmhwildvdnwygfgaradgtskpsyintfqrgeqestwetipqpcwdehkfggqygfld lftkdtgtpakqfkytnapdadaravqatywadqwakeqgksvstsvgkatkmgdylrysffdkyfrkigqpsqagtgyda ahyllswyyawgggidstwswiigsshnhfgyqnpfaawvlstdanfkpkssngasdwaksldrqlefyqwlqsaegaia ggatnswngryeavpsgtstfygmgyvenpvyadpgsntwfgmqvwsmqrvaelyyktgdarakklldkwakwinge ikfnadgtfqipstidwegqpdtwnptqgytgnanlhvkwnygtdlgcasslantltyyaaksgdetsrqnaqklldamwn nysdskgistveqrgdyhrfldqevfvpagwtgkmpngdviksgvkfidirskykqdpewqtmvaalqagqvptqrlhrf waqsefavangvyailfpsmtykvfantatpgdvngdgvvngrdlmelrqylagkldaskinlaaadvnndgvvngrdim eltkliakle

It is however understood that the skilled artisan may introduce additional modifications in the cellulase sequence, such as deletions, substitutions and/or addition of amino acids. It is well known in the art that a polypeptide can be modified by substitution, insertion, deletion and/or addition of one or more amino-acids while retaining its cellulase enzymatic activity. For example, substitution of one amino-acid at a given position by a chemically equivalent amino-acid that does not affect the functional properties of a protein is common. Substitutions may be defined as exchanges within one of the following groups:
■ Small aliphatic, non-polar or slightly polar residues : Ala, Ser, Thr, Pro, Gly
■ Polar, negatively charged residues and their amides : Asp, Asn, Glu, Gln
■ Polar, positively charged residues : His, Arg, Lys
■ Large aliphatic, non-polar residues : Met, Leu, Ile, Val, Cys
■ Large aromatic residues : Phe, Tyr, Trp.

Thus, changes that result in the substitution of one negatively charged residue for another (such as glutamic acid for aspartic acid) or one positively charged residue for another (such as lysine for arginine) can be expected to produce a functionally equivalent product.

The positions where the amino-acids are modified and the number of amino-acids subject to modification in the amino-acid sequence are not particularly limited. The man skilled in the art is able to recognize the modifications that can be introduced without affecting the activity of the protein. For example, modifications in the N- or C-terminal portion of a protein may be expected not to alter the activity of a protein under certain circumstances

Methods for replacing genes and more generally for modifying *Clostridium acetobutylicum* strains are known in the art, particularly disclosed in (Soucaille P. & al , 2006, Heap J.T & al, 2010).

The person skilled in the art shall decide on the most appropriate methods to replace the wild type *cel48A* gene and to transform the strain, prior or after modification, to favor production of a targeted bulk chemical.

In a preferred embodiment, the modified strain is further adapted for an improved growth on cellulose, with the steps of
- repeated subcultures on cellulose of the modified *Clostridium acetobutylicum* and
- selection of the modified strains of *Clostridium acetobutylicum* having an improved ability to grow on cellulose.

Conditions for repeated subcultures are well known to the person skilled in the art (Sonderegger M. and Sauer U., 2003) Selection of the strains having an improved ability to grow on cellulose can be easily done since only the strains able to grow faster on cellulose will also consume faster cellulose and produce faster the bulk chemicals

The method of the invention for the production of a targeted bulk chemical can be performed at different scales, from a laboratory to batches of 400 m3 or more.

Optimized conditions for the pretreatment of cellulose and biomass and the culture of microorganisms are known in the art and disclosed, among others, in Lynd L.R. & al, 2005

In a preferred embodiment the invention provides a process for the continuous production of the targeted bulk chemicals from a recombinant *Clostridium acetobutylicum* comprising: (a) continuously feeding the recombinant organism of the present invention with cellulose or a cellulose containing biomass cultivated in a membrane reactor whereby the cellulosome complex, the undigested cellulose and the microorganism will be retained in the reactor and the water soluble bulk chemical will cross the ultrafiltration membrane (b) recovering the bulk chemical from the cell free ultrafiltrate.

Purification of the targeted bulk chemical is made according to methods known in the art, like for the production of ethanol , butanol , glycerol , 1,2-propanediol , 1,3-propanediol, acetone isopropanol, hydrogen, acetic acid and lactic acid.

### DRAWINGS

**Figure 1** is a schematic representation of plasmid pET-cel9C
**Figure 2** is a schematic representation of plasmid pET-cel9X
**Figure 3** is a schematic representation of plasmid pET-cel48A
**Figure 4** represents the strategy used for fusion by PCR of the Ce148F catalytic and Ce148A dockerin domain coding fragment
**Figure 5** represents the nucleotide sequence of the hybrid Ce148F catalytic and Ce148A dockerin domain coding fragment constructed by PCR fusion (SEQ ID NO 4). Bold underlined refer to linker sequence.
**Figure 6** represents the pET-FH3 plasmid : expression of the CE148FA hybrid protein in E. coli.
**Figure 7** is a schematic representation of plasmid pGR1
**Figure 8** is a schematic representation of plasmid pGR2
**Figure 9** is a schematic representation of plasmid pGR3
**Figure 10** is a schematic representation of plasmid pGR4
**Figure 11** is a schematic representation of plasmid pGR48A-5
**Figure 12** is a schematic representation of the membrane bioreactor for the conversion of cellulose to bulk chemicals: 1 : feed tank containing cellulose ; 2 : continuous N2 sparging ; 3 : reactor ; 4 : cell bleeding ; 5 : gas outlet ; 6 : redox measured ; 7 : pH regulation (ammonium hydroxide solution) ; 8 : cell recycle stream ; 9 : ultra-filtration module ; 10 : excess permeate recycle; 11 cell free cellulose free.

### EXAMPLES

### Example I Cel9C and Cel9X are functional on crystalline cellulose

### -I.1. Ce19C expression

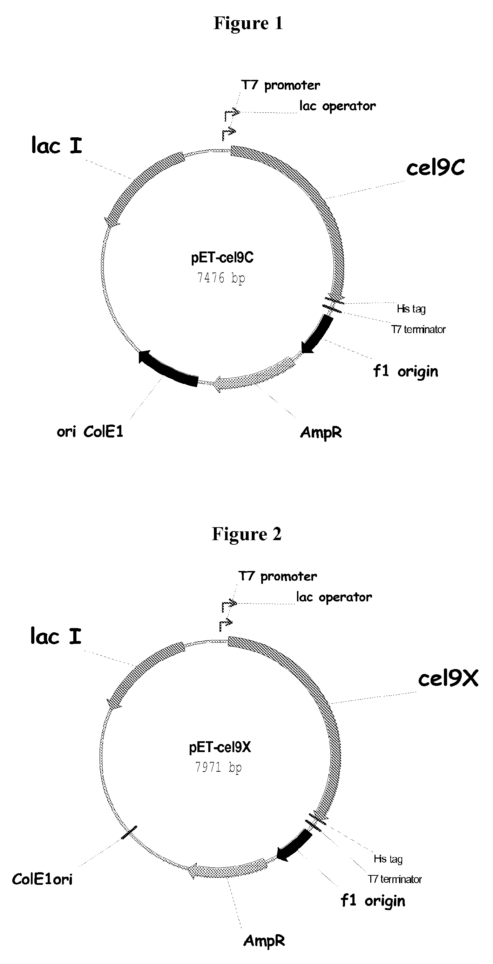

The sequence coding for the mature Ce19C protein was PCR amplified using the genomic DNA from C. *acetobutylicum* ATCC824 as template and the oligonucleotides CelCdir and CelCrev, containing respectively NcoI and XhoI site at their 5'-ends, as primers. These primers were designed in order to amplify only the sequence encoding the mature protein, without the signal peptide .The amplified fragment was then cloned into the pGEMT-Easy vector (Proméga).After checking the integrity of the gene by sequencing, the recombinant plasmid was digested by NcoI and XhoI, and the 2045bp fragment containing the ce19C gene was agarose gel purified and cloned into the pET-22b(+) vector (Novagen), previously digested with the same enzymes, to give the final plasmid pET-ce19C (7476bp) (Fig.1).

In this construct, the sequence coding for the mature Ce19C protein is translationally fused at its 5' end to the sequence coding for the signal peptide PelB functional in E. *coli,* and at its 3' end to the sequence coding for the 6 x His tail. The expression of this hybrid gene is under the control of the T7 promoter.

| **primer** | **SEQ ID N°** | **Séquence (5'-3')** |
|---|---|---|
| CelC_{dir} | 5 | aagtaa***ccatgg***atgaaactaatacaaattttaattatggtgaagcac |
| CelCᵣₑᵥ | 6 | ataa***ctcgag***aggattcacactagaaatctttctgattaactcc |

### -I.2. : Cel9X expression

The sequence coding for the mature Ce19X protein was PCR amplified using the genomic DNA from C. *acetobutylicum* ATCC824 as template and the oligonucleotides CeIX-D2 and CeIX-CT2, containing respectively MscI and XhoI site at their 5'-ends, as primers. These primers were designed in order to amplify only the sequence encoding the mature protein, without the signal peptide. The amplified fragment was then cloned into the pGEMT-Easy vector (Proméga).After checking the integrity of the gene by sequencing, the recombinant plasmid was digested by MscI and XhoI, and the 2541bp fragment containing the ce19X gene was agarose gel purified and cloned into the pET-22b(+) vector (Novagen), previously digested with the same enzymes, to give the final plasmid pET-ce19X (7971bp) (Fig.2).

In this construct, the sequence coding for the mature Ce19X protein is translationally fused at its 5' end to the sequence coding for the signal peptide PelB functional in *E. coli,* and at its 3' end to the sequence coding for the 6 x His tail. The expression of this hybrid gene is under the control of the T7 promoter.

| **primer** | **SEQ ID N°** | **Séquence (5'-3')** |
|---|---|---|
| CeIX-D2 | 7 | aa***tggcca***tgccaaacagcaatgtaggtacacatgatcttattag |
| CeIX-CT2 | 8 | ccg***ctcgag***tttattactttctactggaaaagaagttacctttcctg |

### -I.3. Catalytic properties of Ce19C and Ce19X

The specific activities of Ce19C and Ce19X were studied, using carboxymethyl cellulose (CMC), Phosphoric Acid Swollen Cellulose (PASC) and Avicel (crystalline cellulose) as substrates. The degradation activities were followed for at least two hours. The results are summarized in Table 1 and compared with the activities of homologous Ce19G and Ce19E from C. *cellulolyticum,* which are known to act efficiently on crystalline cellulose (Bélaïch et al., 2002; Gal et al., 1997). Ce19C and Ce19X exhibited similar level of activities than respectively Cel9G and Ce19E from C. *cellulolyticum* on CMC and PASC but also on crystalline cellulose.

**Table A. Activity of Ce19C and Ce19X from C. acetobutylicum on various substrates and comparison with activities of previously characterized family 9 cellulases from C. cellulolyticum.**

| **Substrate** | **Activity (IU/µM)** | | | |
|---|---|---|---|---|
| | ***C. acetobutylicum*** | | ***C. cellulolyticum*** | |
| | **Ce19C** | **Ce19X** | **Ce19G** | **Ce19E** |
| CMC | 1217 | 30.2 | 1170 | 13.5 |
| PASC | 25.8 | 36.7 | 38 | 42.5 |
| Avicel | 2.6 | 2.1 | 5 | 5.8 |

### Example II Cel48A is not functional

### -II.1 : Cel48A expression

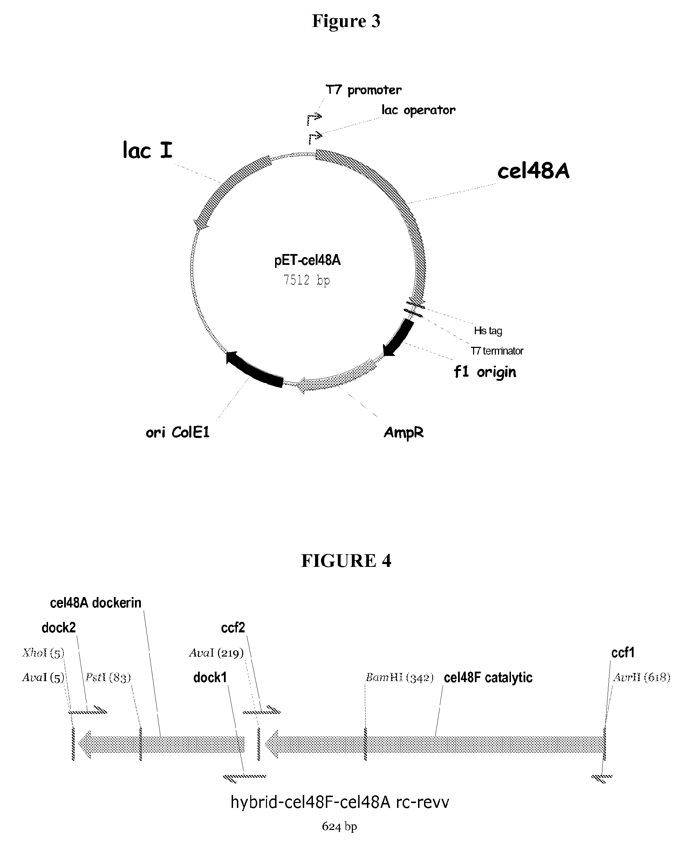

The sequence coding for the mature Ce148A protein was PCR amplified using the genomic DNA from C. *acetobutylicum* ATCC824 as template and the oligonucleotides Ce1A-Nter and Ce1A-Cter, containing respectively NcoI and XhoI site at their 5'-ends, as primers. These primers were designed in order to amplify only the sequence encoding the mature protein, without the signal peptide. The amplified fragment was then cloned into the pGEMT-Easy vector (Proméga). After checking the integrity of the gene by sequencing, the recombinant plasmid was digested by NcoI and XhoI, and the 2081bp fragment containing the ce148A gene was agarose gel purified and cloned into the pET-22b(+) vector (Novagen), previously digested with the same enzymes, to give the final plasmid pET-ce148A (7512bp) (Fig.3).

In this construct, the sequence coding for the mature Ce148A protein is translationally fused at its 5' end to the sequence coding for the signal peptide PelB functional in *E. coli,* and at its 3' end to the sequence coding for the 6 x His tail. The expression of this hybrid gene is under the control of the T7 promoter.

| **primer** | **SEQ ID N°** | **Séquence (5'-3')** |
|---|---|---|
| Ce1A-Nter | 9 | ctaataaag***ccatggc***tgctacaactacagattcatcc |
| Ce1A-Cter | 10 | ccg***ctcgag***ttttgcaattaatttagtaagttcc |

### -I.3. Catalytic properties of Cel48A

Native Ce148A showed very low activities on both CMC and PASC, (Table. B). No enzyme activity was observed on crystalline cellulose (Avicel).

**Table B. Enzyme activities (IU/µM) recombinant Cel48A protein compare between wild type and recombinant.**

| **substrate** | **Specific activity (IU/µM)** | |
|---|---|---|
| | ***C. acetobutylicum*** | ***C. cellulolyticum*** |
| | **Ce148A** | **Ce148F** |
| **CMC** | 0.011 | ND |
| **PASC** | 0.0020 | 42.5 |
| **Avicel** | ND | 13.4 |

| | | |
|---|---|---|
| ND not detected | | |

### Example III. Construction of a functional hybrid protein with a family 48 glycoside hydrolase active catalytic domain and a cel48A dockerin domain

As we demonstrated above that Cel48A has a nonfunctional catalytic domain, we aim to replace this domain by the family 48 glycoside hydrolase catalytic domain from the Cel48F cellulase of C. *cellulolyticum* while keeping the Cel48A dockerin domain for the binding to the CipA scaffolding protein of C. *acetobutylicum* We named this chimera protein Cel48FA

### -1. Hybrid gene construction

The Ce148FA protein is an hybrid protein containing the catalytic domain of Ce148F from *Clostridium cellulolyticum* and the dockerin domain of Ce148A from *Clostridium acetobutylicum.*

In order to design the hybrid gene, alignment of aa sequences of Ce148A and Ce148F was analysed and allowed starting point of dockerin domain of Ce148A identification. Normally, C. *acetobutylicum* dockerin domains contain a duplicated sequence of about 22 aa residues, the first 12 of which are homologous to the well known structure of the calcium-binding loop in the EF-hand motif (Sabathé *et al.,* 2002). The end point of catalytic domain of Cel48F derived from previous results from Fierobe *et al.,* 2001. By the way, the end of the catalytic domain is very close to the start point of the dockerin domain.

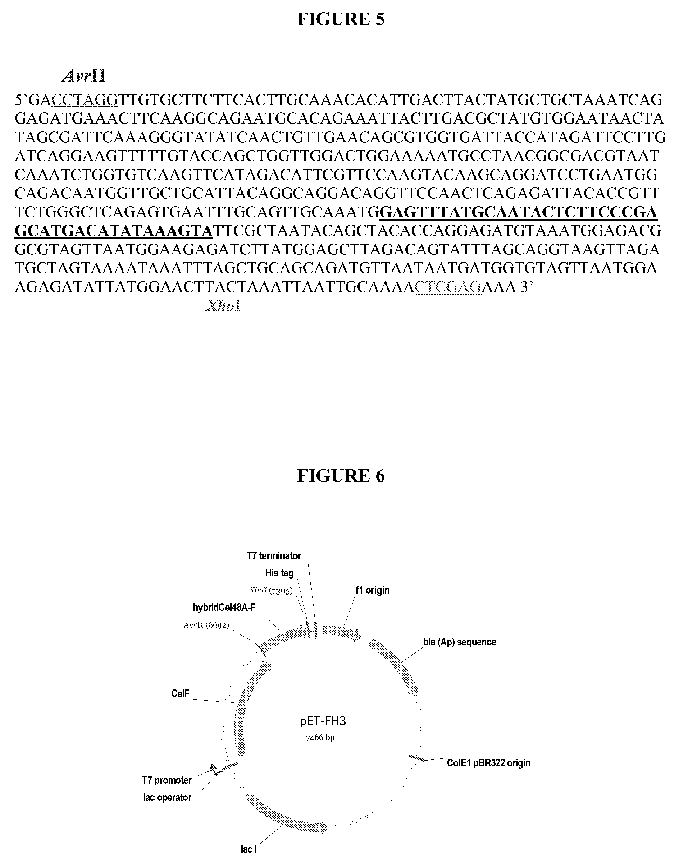

The chimeric gene coding for the Cel48FA protein was constructed by PCR fusion (Figure 5) The 3'-end of the sequence coding for the catalytic domain of Cel48F was PCR amplified using the pET-Fc plasmid (Reverbel-leroy *et al.,* 1996) as template and the oligonucleotides ccf1 and ccf2 as primers. The ccf1 primer encompasses the AvrII site of the cel48F WT gene.
On the other hand, the sequence coding for the dockerin domain of Cel48A was PCR amplified using the genomic DNA from C. *acetobutylicum* as template and the oligonucleotides dock1 and dock2 (containing a XhoI site at the 5'-end) as primers.
As the ccf2 and dock1 primers have self-complementary 5'-regions, the two sequences were then PCR fused using the ccf1 and dock2 primers (Figure 4). This fusion PCR product was then AvrII+XhoI digested and the 613bp fragment obtained was ligated with the large fragment of the pET-Fc plasmid digested with the same enzymes to give the resulting plasmid pET-FH3 (Figure 6).

In this construction, the 3'-end of the sequence coding for the hybrid protein Ce148FA is fused to a sequence coding for a 6xHis tag, and the expression of this chimera gene is placed under the control of the T7 promoter.

This plasmid was then introduced in *E. coli* BL21 CodonPlusRIL strain. This strain contains plasmids encoding rare tRNA to enhance efficiency of heterologous protein production in *E. coli.* The hybrid protein was overexpressed in a soluble form and purified by Ni-NTA affinity chromatographic.

### -2. Activity assay

Enzyme activities, were assayed using purified and concentrated soluble form of recombinant enzyme. Native Ce148A showed very low activities on both CMC and PASC, (Table. C), whereas no enzyme activity could be observed on crystalline cellulose. In contrast, the purified hybrid Ce148FA showed proficient activity on all cellulosic substrates tested. Interestingly, the hybrid protein shows activity on crystalline cellulose as good as the native Ce148F enzyme(11.88 and 13.4 IU/µmol respectively).

| **primer** | **SEQ ID NO** | **Séquence (5'-3')** |
|---|---|---|
| ccf-1 | 11 | ga***cctagg***ttgtgcttcttca |
| ccf-2 | 12 | tactttatatgtcatgctcgggaagagtattgcataaactc |
| dock-1 | 13 | ttcccgagcatgacatataaagtattcgctaatacagctacaccagg |
| dock-2 | 14 | ttt***ctcgag***ttttgcaattaatttagtaagttccataatatc |

**Table C. Enzyme activities (IU/µM) recombinant Cel48A protein compare between wild type and recombinant.**

| **Substrate** | **Specific activity (IU/µM)** | | |
|---|---|---|---|
| | ***C. acetobutylicum*** | | ***C. cellulolyticum*** |
| | **CelFA48 hybrid** | **Cel48A** | **Cel48F** |
| **CMC** | 0.34 | 0.011 | ND |
| **PASC** | 55.42 | 0.0020 | 42.5 |
| **Avicel** | 11.88 | - | 13.4 |

### Example IV:

### Restoration of an active cellulososme by chromosmic replacement of the defective cel48A gene by the functional hybrid gene cel48SAFA

In order to replace, without marker left, the defective *cel48A* gene by the functional hybrid gene *cel48SAFA,* a new method was developed based on i) a markerless replacement cassette ii) a non replicative plasmid for initial single crossing over selection and ii) the use of the upp gene for the positive selection of the second crossing over event. For this purpose the pGR48A-5 plasmid was constructed in five steps described below and then use to repair the cellulosome of C. *acetobutylicum.* After replacement on the chromosome of *cel48A* native gene by *cel48SAFA* gene, this latter gene will be in the same genetic background as the native gene, and its transcriptional regulation under the control of the natural *cel* cluster promoter, located upstream the *cip*A gene

### 1- Construction of pGR 1

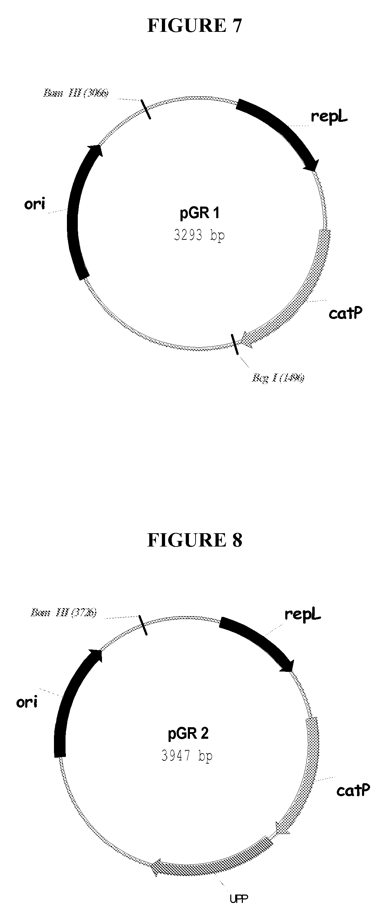

This plasmid contains a pIM13 origin of replication functional in Clostridia (rolling circle mechanism of replication), a *cat*P gene conferring resistance to thiamphenicol and a unique *Bam*HI site for the cloning of the replacement cassette. pGR1 was constructed in a two steps process from the pETSPO plasmid (Harris et al, 2002, J. Bacteriol) to remove part of a polylinker and among others a *Bam*HI and an *Eco*RI sites. IPCR was performed with the Pwo DNA polymerase with pETSPO as a template plasmid and oligonucleotides PGRAccI and PGREcoRI as primers, and the PCR product was phosphorylated then ligated. After transformation *of E. coli,* the pGR0 plasmid was obtained. This plasmid was then digested with *Bam*HI to remove the *spo0A* cassette and the proper DNA fragment purified and self-ligated to obtain the pGR1 plasmid. The map of pGR1 is given in figure 7.

### 2- Construction of the pGR 2

The *upp* gene with its own Ribosome Binding Site **(RBS)**was cloned into pGR1 at the *BcgI* site just after the *CatP* gene in order to construct an artificial operon with *upp* expressed under the control of the *CatP* promotor. In this way, no homologous regions was introduced which would allow chromosomal integration of *UPP* gene in the Δ*cac15*Δ*upp* strain in further deletion experiments using the *upp* gene as a counter selectable marker for plasmid loss.

The *UPP* gene with its **RBS** was PCR (Pfu) amplified on genomic DNA from C. *acetobutylicum* using oligonucleotides REP-UPP F et REP-UPP R as primers. The 664 bp PCR-product was digested by *Pvu*II and cloned into pGR1 digested by *Bcg*I and treated with T4 DNA Polymerase for blunting ends. After transformation of *E. coli,* the clones containing the recombinant plasmid with the *upp* gene in the same orientation as that of catP gene, creating an artificial operon under the control of the *CatP* promotor were screened by colonies PCR using the primer CatP3'-D and REP-UPP R. The pGR 2 (cf. figure 8) replicative vector was obtained.

### 3- Construction of the pGR 3

Two DNA sequences surrounding the *cel48A* (CA_C 911) were PCR amplified on genomic DNA from C. *acetobutylicum* ATCC824 with two couples of primers CelA-P+Ce1A-2 (upstream region of cel48A) and CelA-3+CelA-4 (downstream region of ce148A) giving 764bp and 1142bp long size products respectively.
Each primer CelA-P and CelA-4 introduces a BamHI site, while primers CelA-2 and CelA-3 have self-complementary 5'-regions which introduce a StuI site. DNA fragments CelA-P+CelA-2 and CelA-3+CelA-4 are joined in a fusion PCR with primers CelA-P and CelA-4.

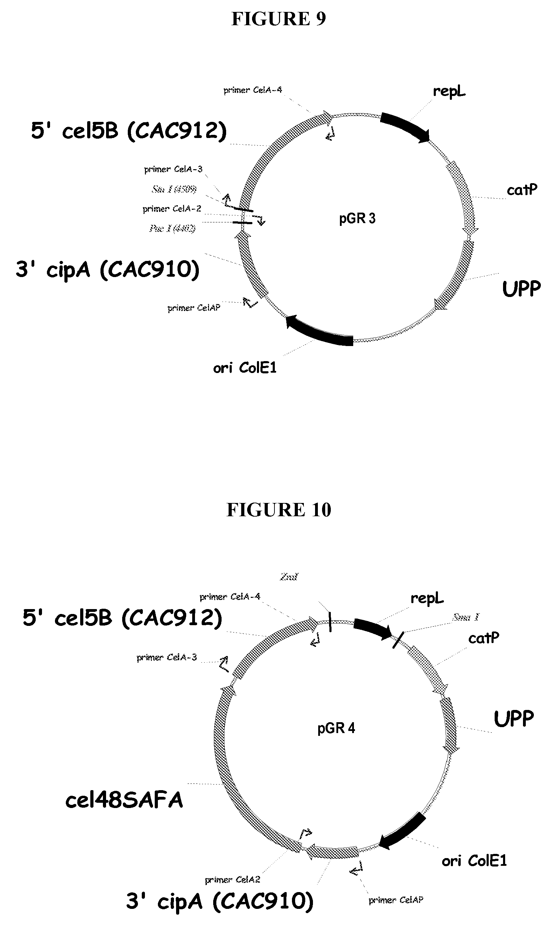

This PCR fusion product was then *Bam*HI digested and inserted into the PGR 2 plasmid previously digested by *Bam*HI to give the pGR 3 plasmid (Fig. 9)

### 4- Construction of the pGR 4

The CEL48SAFA protein is an hybrid protein containing the signal peptide of Cel48A from *Clostridium acetobutylicum.*ATCC824*,* the catalytic domain of Cel48F from *Clostridium cellulolyticum,* and the dockerin domain of Ce148A from *Clostridium acetobutylicum* ATCC824.

The chimeric gene coding for the Ce148SAFA protein was constructed as follows : the sequence encoding the Cel48FA protein was PCR amplified using the using the pET-FH3 plasmid (see example III) as template and the oligonucleotides SAFA1-D and SAFA1-R as primers, both containing *Pvu*I sites at their 5'-ends. The SAFA1-D primer contains in its 5' end the sequence coding for the signal sequence and the RBS of the native Ce148A from *Clostridium acetobutylicum* ATCC824.

This PCR product was then *Pvu*I digested and ligated with the PGR 3 plasmid previously digested by *Pac*I*.* After transformation of *E. coli,* the clones containing the recombinant plasmid with the *cel48*SAFA gene in the correct orientation, ie in the same as the 3'-end of the cipA gene were screened by colonies PCR using the primer CelA-P and ccf2 as primers. The correct plasmid was named pGR4 (Fig. 10). In this construct, the sequence coding for the hybrid Ce148SAFA protein is translationally fused at its 3' end to the sequence coding for the 6 x His tail

### 5- Construction of the pGR48A-5 plasmid

Finally, in order to obtain a non replicative vector, to be used as suicide vector for single crossing over integration in the *cel48A* locus on the C. *acetobutylicum* chromosome, the final step was to remove from the pGR4 plasmid the clostridial origin of replication *rep*L*.*

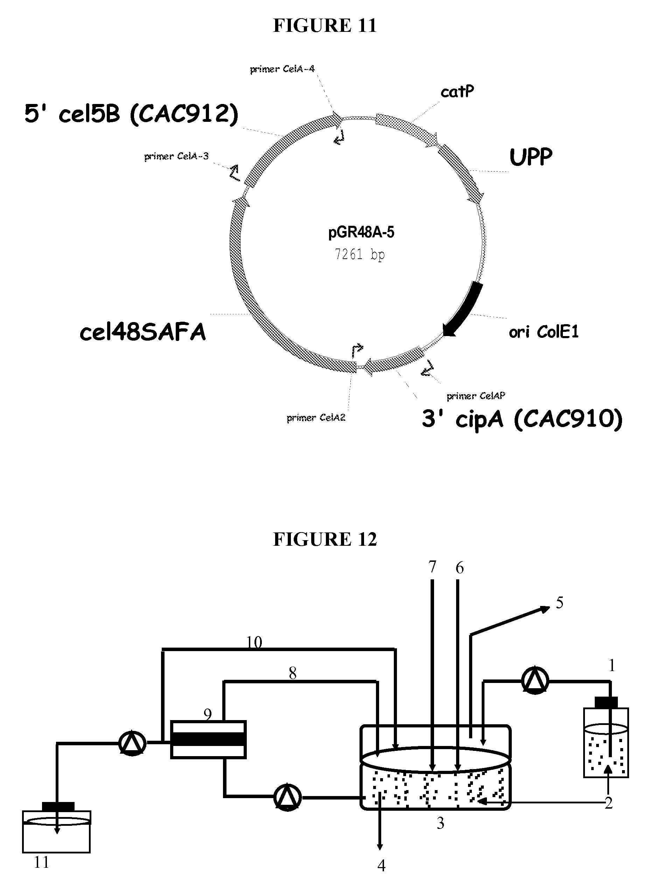

The pGR4plasmid was thus *Sma*I and *Zra*I digested, and the large fragment obtained was agarose gel purified and self ligated, to give the final pGR48A-5 plasmid (, Fig. 11).

### 6- Replacement of cel48A by ce148SAFA

The pGR48A-5 suicide plasmid was used to transform by electroporation C. *acetobutylicum* Δ*cacl5*Δ*upp* strain (WO2008/060387). This strain is the WT strain with deletions of the restriction system *cac824I* gene and *upp* gene encoding uracil phospho- ribosyl transferase and is thus MLS^{s}, Thiam^{s} and 5-FU^{R} and its transformation doesn't necessitate prior *in vivo* methylation of the plasmid.

Integrants were selected on RCA with thiamphenicol at 10 µg/ml. . Integration of the plasmid by single crossing over was demonstrated to occur mainly at the *cel5B* locus. Cells from one one of those recombinant strains were resuspended in RCM and spread on RCA with 5-FU 400 µM. The correct phenotype of the clones growing on these plates was checked by their sensitivity to thiamphenicol 10µg/mL; As the second crossing over can lead to either the integration of *celSAFA* or to wild type genotype the clones were analyzed by PCR. About 10% of the clones have the correct genotype namely a replacement of *cel48A* by *cel48SAFA.* These clones were then checked for their ability to grow on minimal medium plate containing 0.5% PASC. Colonies appears on the plates after a week at 35°C while no colonies were obtained with the control strain..

The recombinant strain was named C. *Acetobutylicum* SAFA.

| **primer** | **SEQ ID N°** | **Séquence (5'-3')** |
|---|---|---|
| PGRAccI | 15 | ccggggtaccgtcgacctgcagcc |
| PGREco RI | 16 | gaattccgcgagctcggtacccggc |
| REP-UPP F | 17 | aaaacagctgggaggaatgaaataatgagtaaagttacac |
| REP-UPP R | 18 | aaaacagctgttattttgtaccgaataatctatctccagc |
| CatP3'-D | 19 | ggaaggaaagccaaatgctccgg |
| CelA-P | 20 | gttcttaa***ggatcc***aagtgattacacagtaacaagtgatgg |
| CelA-2 | 21 | gggg***aggcct***aaaaagggggaaatataactgtagatgtaagagctacagcc |
| CelA-3 | 22 | cccccttttt***aggcct***ccccttagctgcagcagatgttaataatgatggtg |
| CelA-4 | 23 | aaaa***ggatcc***tcttgtatatagaaccagcttggacgcc |
| SAFA1-D-PvuI | 24 | aaacgatcgaggagatagaaaaatatgttaaagataagtaagaattttaaaaaaataatggctgtagct cttacatctacagttatatttggttctctatctggattattaactaataaagttgcagctgctacaactacag caaacaaggtgtaccaggatcg |
| SAFA1-R-PvuI | 25 | ttttcgatcgttatcagtggtggtggtggtggtgc |
| ccf-2 | 26 | tactttatatgtcatgctcgggaagagtattgcataaactc |

### EXAMPLE V Adaptative evolution for better growth on cellulose.

The C. *acetobutylicum* SAFA strain was initially grown in anaerobic flask culture in the synthetic medium described by Soni et al (Soni et al, 1987, Appl. Microbiol.Biotechriol. 27:1-5) supplemented with 2.5 g/l of ammonium acetate and with a replacement of glucose by 5 g/l of PASC. When incubated at 35 °C, growth on PASC was observed during the fist 3 days but it was followed by a cell lysis phase. Good solubilization of PASC could be visually observed after a week.

In order to evolve the strain for faster growth on cellulose, we made serial subcultures every 3 days in the PASC synthetic medium described above. After the 8th subculture, all the PASC was solubilized after 3 days and we had to make subcultures every 2 days. After the 15th subculture we got an evolved population that could completely solubilized 5g/l PASC in 2 days and reach an optical density at 620nm of 1.1 .

### EXAMPLE VI Membrane bioreactor for continuous production of bulk chemicals from cellulose.

In order to efficiently produce a targeted bulk chemicals in a continuous process using a recombinant cellulolytic C. *acetobutylicum,* we develop the following reactor comprising a continuous fed with cellulose or a cellulose containing biomass feeding a membrane reactor whereby the microorganism, the cellulosome complex and the undigested cellulose will be retained in the reactor and the water soluble bulk chemical will cross the ultrafiltration membrane (Figure 12).

Continuous cultures in cell recycle membrane bioreactor (figure 12) were carried out in a 600 ml glass reactor (Midiverre, France) ( 3) connected to an ultra-filtration flat module (KeRAM INSIDE, TAMI France, molecular weight cut-off 50 KD, surface area 0.25 m2) (9). The fermentation broth was recirculated in the membrane by peristaltic pump (Masterflex Industrial, Fischer Bioblock Scientific) (8). The total culture volume was 450 ml, the pH and the temperature were regulated respectively at 5 (ammonium hydroxide) (7) and 35°C. The medium is the synthetic medium described by Soni et al (Soni et al, 1987, Appl. Microbiol.Biotechnol. 27:1-5) with 30 g/l PASC replacing glucose. Nitrogen was continuously sparged in the reactor. After a 10 % inoculation with the evolved population described in example 5, the cell recycle membrane bioreactor was operated in batch mode for 24 hours before switching to continuous mode with total cell recycle at a dilution rate of permeate of 0.2 h-1. When PASC limitation was reached, a cell bleeding rate was applied at 0.002 h-1.

Under steady state condition, all the PASC was consumed and converted to a mixture of butanol, acetone, ethanol, acetic acid and butyric acid.

**TABLE 1**

| **Sequences encoding dockerins domains that could bind to the cohesin domains of the *Clostridium acetobutylicum* CipA scaffolding protein** |
|---|
| |
| NP 347547.1 |
| processive endoglucanase [Clostridium acetobutylicum ATCC 824] >gb\|AAK78887.1\|AE007607_1 Possible processive endoglucanase family 48, secreted; CelF ortholog; dockerin domain [Clostridium acetobutylicum ATCC 824] |
| NP 347551.1 |
| endoglucanase A [Clostridium acetobutylicum ATCC 824] >gb\|AAK78891.1\|AE007607_5 Endoglucanase A precursor (endo-1,4-beta-glucanase) (cellulase A), secreted; dockerin domain [Clostridium acetobutylicum ATCC 824] |
| NP 347554.1 |
| non-processive endoglucanase [Clostridium acetobutylicum ATCC 824] >gb\|AAK78894.1 \|AE007607_8 Possible non-processive endoglucanase family 5, ortholog of mannase A, secreted; dockerin domain [Clostridium acetobutylicum ATCC 824] |
| NP 347549.1 |
| non-processive endoglucanase [Clostridium acetobutylicum ATCC 824] >gb\|AAK78889.1 \|AE007607_3 Possible non-processive endoglucanase family 9, secreted; Ce1G ortholog; dockerin and cellulose-binding domain [Clostridium acetobutylicum ATCC 824] |
| NP 347552.1 |
| non-processive endoglucanase [Clostridium acetobutylicum ATCC 824] >gb\|AAK78892.1 \|AE007607_6 Possible non-processive endoglucanase family 9, secreted; CelG ortholog; dockerin and cellulose-binding domain [Clostridium acetobutylicum ATCC 824] |
| NP 347548.1 |
| non-processive endoglucanase [Clostridium acetobutylicum ATCC 824] >gb\|AAK78888.1 \|AE007607_2 Possible non-processive endoglucanase family 5, secreted; CelA homolog secreted; dockerin domain [Clostridium acetobutylicum ATCC 824] |
| NP 347553.1 |
| glycoside hydorlase [Clostridium acetobutylicum ATCC 824] >gb\|AAK78893.1\|AE007607_7 and cellulose-binding endoglucanase family 9; CelL ortholog; dockerin domain [Clostridium acetobutylicum ATCC 824] |
| NP 347200.1 |
| cellulase CelE-like protein [Clostridium acetobutylicum ATCC 824] >gb\|AAK78540.1\|AE007571_6 Cellulase CelE ortholog; dockerin domain [Clostridium acetobutylicum ATCC 824] |
| NP 347555.1 |
| sialidase [Clostridium acetobutylicum ATCC 824] >gb\|AAK78895.1\|AE007608_1 Probably secreted sialidase; several ASP-boxes and dockerin domain [Clostridium acetobutylicum ATCC 824] |
| NP 350057.1 |
| endoglucanase family protein [Clostridium acetobutylicum ATCC 824] >gb\|AAK81397.1\|AE007844_7 Endoglucanase family 5; S-layer homology, cell-adhesion and dockerin domains [Clostridium acetobutylicum ATCC 824] |

**TABLE 2**

| **Sequences encoding family 48 glycoside hydrolases** | |
|---|---|
| **Accession** | **Description** |
| ZP 05497269.1 | glycoside hydrolase family 48 [Clostridium papyrosolvens DSM 2782] >gb\|EEU57778.1\| glycoside hydrolase family 48 [Clostridium papyrosolvens DSM 2782] |
| BAA32430.1 | exoglucanase [Clostridium josui] >dbj\|BAI52924.1\| cellulase [Clostridium josui] glycoside hydrolase family 48 [Clostridium cellulovorans 743B] |
| YP 003844283.1 | >gb\|ADL52519.11 glycoside hydrolase family 48 [Clostridium cellulovorans 743B] glycoside hydrolase family 48 [Clostridium cellulolyticum H10] >sp\|P37698.2\|GUNF_CLOCE RecName: Full=Endoglucanase F; AltName: Full=Cellulase F; |
| YP 002505088.1 | AltName: Full=EGCCF; AltName: Full=Endo-1,4-beta-glucanase F; Flags: Precursor >gblAAB41452.1\| cellulase precursor [Clostridium cellulolyticum H10] > gb\|ACL75108.1 glycoside hydrolase family 48 [Clostridium cellulolyticum H10] |
| AAC38571.3 | exoglucanase S [Clostridium cellulovorans] RecName: Full=Exoglucanase-2; AltName: Full=1,4-beta-cellobiohydrolase II; AltName: Full=Avicelase II; AltName: |
| P50900.1 | Full=Exocellobiohydrolase II; AltName: Full=Exoglucanase II; Flags: Precursor >emb\|CAA93280.1\| avicelase II [Clostridium stercorarium] cellulose 1,4-beta-cellobiosidase [Clostridium phytofermentans ISDg] |
| YP 001560460.1 | >gb\|ABX43721.1\| Cellulose 1,4-beta-cellobiosidase [Clostridium phytofermentans ISDg] Cellulase., Cellulose 1,4-beta-cellobiosidase [Caldicellulosiruptor obsidiansis |
| YP 003840939.1 | OB47] >gb\|ADL42953.1\| Cellulase., Cellulose 1,4-beta-cellobiosidase [Caldicellulosiruptor obsidiansis OB47] Cellulose 1,4-beta-cellobiosidase [Caldicellulosiruptor obsidiansis OB47] |
| YP 003840931.1 | >gb\|ADL42945.1\| Cellulose 1,4-beta-cellobiosidase [Caldicellulosiruptor obsidiansis OB47] glycoside hydrolase family protein [Caldicellulosiruptor saccharolyticus DSM |
| YP 001179881.1 | 8903] >gb\|ABP66690.1\| glycoside hydrolase, family 48 [Caldicellulosiruptor saccharolyticus DSM 8903] RecName: Full=Endoglucanase A; AltName: Full=Cellulase A; AltName: |
| P22534.2 | Full=Endo-1,4-beta-glucanase A; Flags: Precursor >gb\|AAA91086.1\| cellulase [Caldicellulosiruptor saccharolyticus] glycoside hydrolase family 48 [Caldicelulosiruptor becscii DSM 6725] |
| YP 002573718.1 | >gb\|ACM60945.1\| glycoside hydrolase family 48 [Caldicelulosiruptor becscii DSM 6725] glycoside hydrolase family 48 [Caldicelulosiruptor becscii DSM 6725] |
| YP 002573728.1 | >gb\|ACM60955.1\| glycoside hydrolase family 48 [Caldicelulosiruptor becscii DSM 6725] glycoside hydrolase family 48 [Caldicelulosiruptor becscii DSM 6725] |
| YP 002573721.1 | >gb\|ACM60948.1\| glycoside hydrolase family 48 [Caldicelulosiruptor becscii DSM 6725] |
| CAB06786.1 | 1,4-beta-glucanase [Anaerocellum thermophilum] glycoside hydrolase family 48 [Clostridium lentocellum DSM 5427] |
| ZP 06884220.1 | >gb\|EFG98418.1\| glycoside hydrolase family 48 [Clostridium lentocellum DSM 5427] cellulose 1,4-beta-cellobiosidase [Streptosporangium roseum DSM 43021] |
| YP 003336683.1 | >gb\|ACZ83940.1\| cellulose 1,4-beta-cellobiosidase [Streptosporangium roseum DSM 43021] cellulose 1,4-beta-cellobiosidase [Streptomyces avermitilis MA-4680] |
| NP 823031.1 | >dbj\|BAC69566.1\| putative secreted cellulose 1,4-beta-cellobiosidase [Streptomyces avermitilis MA-4680] |
| ADI10021.1 | cellulose 1,4-beta-cellobiosidase [Streptomyces bingchenggensis BCW-1] secreted cellulase [Streptomyces lividans TK24] >ref\|ZP_06527249.1\| secreted |
| ZP 05522420.1 | cellulase [Streptomyces lividans TK24] >gb\|EFD65499.1\| secreted cellulase [Streptomyces lividans TK24] |
| ZP 06922079.1 | cellulose 1,4-beta-cellobiosidase [Streptomyces sviceus ATCC 29083] >gb\|EDY54783.1\| cellulose 1,4-beta-cellobiosidase [Streptomyces sviceus ATCC 29083] secreted cellulase [Streptomyces coelicolor A3(2)] >emb\|CAA20643.1\| putative |
| NP 630627.1 | secreted cellulase [Streptomyces coelicolor A3(2)] cellulose 1,4-beta-cellobiosidase [Streptomyces sviceus ATCC 29083] |
| ZP 06920984.1 | >gb\|EDY60084.1\| cellulose 1,4-beta-cellobiosidase [Streptomyces sviceus ATCC 29083] |
| ZP 05517573.1 | cellulose 1,4-beta-cellobiosidase [Streptomyces hygroscopicus ATCC 53653] cellulose 1,4-beta-cellobiosidase [Streptomyces hygroscopicus ATCC 53653] |
| ZP 07297524.1 | >gb\|EFL25893.1\| cellulose 1,4-beta-cellobiosidase [Streptomyces hygroscopicus ATCC 53653] |
| ZP 05537512.1 | secreted cellulase [Streptomyces griseoflavus Tu4000] cellulose 1,4-beta-cellobiosidase [Streptomyces griseoflavus Tu4000] |
| ZP 07309616.1 | >gb\|EFL37985.1\| cellulose 1,4-beta-cellobiosidase [Streptomyces griseoflavus Tu4000] |
| ZP 04684734.1 | secreted cellulase [Streptomyces ghanaensis ATCC 14672] secreted cellulase [Streptomyces ghanaensis ATCC 14672] >gb\|EFE65975.1\| |
| ZP 06575514.1 | secreted cellulase [Streptomyces ghanaensis ATCC 14672] glycoside hydrolase family 48 [Streptomyces flavogriseus ATCC 33331] |
| ZP 05800968.1 | >gb\|EEW75589.1\| glycoside hydrolase family 48 [Streptomyces flavogriseus ATCC 33331] |
| YP 003494466.1 | putative cellulase [Streptomyces scabiei 87.22] >emb\|CBG75943.1\| putative secreted cellulase [Streptomyces scabiei 87.22] secreted cellulase [Streptomyces viridochromogenes DSM 40736] |
| ZP 07307648.1 | >gb\|EFL36017.1\| secreted cellulase [Streptomyces viridochromogenes DSM 40736] |
| ZP 05535382.1 | secreted cellulase [Streptomyces viridochromogenes DSM 40736] glycoside hydrolase family 48 [Streptomyces sp. ACTE] >gb\|EFB66662.1\| |
| ZP 06273096.1 | glycoside hydrolase family 48 [Streptomyces sp. ACTE] cellulose 1,4-beta-cellobiosidase [Streptomyces sp. e14] >gb\|EFF93968.1\| |
| ZP 06710846.1 | cellulose 1,4-beta-cellobiosidase [Streptomyces sp. e14] glycoside hydrolase family protein [Herpetosiphon aurantiacus ATCC 23779] |
| YP 001543075.1 | >gb\|ABX02947.1\| glycoside hydrolase family 48 [Herpetosiphon aurantiacus ATCC 23779] cellulose 1,4-beta-cellobiosidase [Streptomyces pristinaespiralis ATCC 25486] |
| ZP 06907852.1 | >gb\|EDY67117.1\| cellulose 1,4-beta-cellobiosidase [Streptomyces pristinaespiralis ATCC 25486] |
| YP 003487398.1 | putative cellulase [Streptomyces scabiei 87.22] >emb\|CBG68833.1\| putative secreted cellulase [Streptomyces scabiei 87.22] glycoside hydrolase family 48 [Clostridium thermocellum DSM 2360] |
| ZP 05429046.1 | >gb\|EEU02081.1\| glycoside hydrolase family 48 [Clostridium thermocellum DSM 2360] glycoside hydrolase family protein [Clostridium thermocellum ATCC 27405] >sp\|A3DH67.1\|GUNS_CLOTH RecName: Full=Endoglucanase SS; Short=EGSS; AltName: Full=Cellulase SS; AltName: Full=Endo-1,4-beta-glucanase; Flags: Precursor >sp\|P0C2S5.1\|GUNS_CLOTM RecName: Full=Endoglucanase SS; |
| YP 001038489.1 | Short=EGSS; AltName: Full=Cellulase SS; AltName: Full=Endo-1,4-beta-glucanase; Flags: Precursor >gb\|AAA23226.1\| cellulase [Clostridium thermocellum] >gb\|ABN53296.1\| glycoside hydrolase, family 48 [Clostridium thermocellum ATCC 27405] glycoside hydrolase family 48 [Clostridium thermocellum JW20] |
| ZP 06250120.1 | >gb\|EFB37513.1\| glycoside hydrolase family 48 [Clostridium thermocellum JW20] family 48 glycoside hydrolase [Thermobispora bispora DSM 43833] |
| YP 003652742.1 | >gb\|ADG88849.1\| glycoside hydrolase family 48 [Thermobispora bispora DSM 43833] |
| ACZ98592.1 | cellobiohydrolase [Cellulosilyticum ruminicola] RecName: Full=Exoglucanase B; AltName: Full=1,4-beta-cellobiohydrolase B; |
| P50899.1 | AltName: Full=CBP120; AltName: Full=Exocellobiohydrolase B; Flags: Precursor >gb\|AAB00822.1\| beta-1,4-cellobiohydrolase [Cellulomonas fimi] |
| AAR23324.1 | cellulosomal family-48 processive glycoside hydrolase precursor [Bacteroides cellulosolvens ATCC 35603] glycoside hydrolase family protein [Acidothermus cellulolyticus 11B] |
| YP 872376.1 | >gb\|ABK52390.1\| cellulose 1,4-beta-cellobiosidase [Acidothermus cellulolyticus 11B] cellulose 1,4-beta-cellobiosidase [Amycolatopsis mediterranei U32] |
| YP 003767358.1 | >gb\|ADJ46956.1\| cellulose 1,4-beta-cellobiosidase [Amycolatopsis mediterranei U32] glycoside hydrolase family 48 [Catenulispora acidiphila DSM 44928] |
| YP 003114349.1 | >gb\|ACU72508.1\| glycoside hydrolase family 48 [Catenulispora acidiphila DSM 44928] |
| BAE94321.1 | active phase-associated protein II [Gastrophysa atrocyanea] glycoside hydrolase family 48 [Jonesia denitrificans DSM 20603] |
| YP 003161093.1 | >gb\|ACV08790.1\| glycoside hydrolase family 48 [Jonesia denitrificans DSM 20603] glycoside hydrolase family 48 [Xylanimonas cellulosilytica DSM 15894] |
| YP 003325742.1 | >gb\|ACZ30184.1\| glycoside hydrolase family 48 [Xylanimonas cellulosilytica DSM 15894] |
| BAE94320.1 | active phase-associated protein I [Gastrophysa atrocyanea] |
| ZP 07327526.1 | glycoside hydrolase family 48 [Acetivibrio cellulolyticus CD2] >gb\|EFL61179.1\| glycoside hydrolase family 48 [Acetivibrio cellulolyticus CD2] glycoside hydrolase family 48 [Micromonospora sp. ATCC 39149] |
| ZP 04604703.1 | >gb\|EEP70633.1\| glycoside hydrolase family 48 [Micromonospora sp. ATCC 39149] |
| ZP 06402205.1 | glycoside hydrolase family 48 [Micromonospora sp. L5] >gb\|EFC58399.1\| glycoside hydrolase family 48 [Micromonospora sp. L5] glycoside hydrolase family 48 protein [Micromonospora aurantiaca ATCC 27029] |
| YP 003837256.1 | >gb\|ADL47680.1\| glycoside hydrolase family 48 [Micromonospora aurantiaca ATCC 27029] glycoside hydrolase family protein [Salinispora arenicola CNS-205] |
| YP 001537978.1 | >gb\|ABV98987.1\| glycoside hydrolase family 48 [Salinispora arenicola CNS-205] |
| ZP 07328318.1 | glycoside hydrolase family 48 [Acetivibrio cellulolyticus CD2] >gb\|EFL60351.1\| glycoside hydrolase family 48 [Acetivibrio cellulolyticus CD2] glycoside hydrolase family 48 [Actinosynnema mirum DSM 43827] |
| YP 003099958.1 | >gb\|ACU36112.1\| glycoside hydrolase family 48 [Actinosynnema mirum DSM 43827] |
| CAH25542.1 | cellulose 1,4-beta-cellobiosidase [Otiorhynchus sulcatus] glycoside hydrolase family protein [Salinispora tropica CNB-440] |
| YP 001159773.1 | >gb\|ABP55395.1\| cellulose 1,4-beta-cellobiosidase [Salinispora tropica CNB-440] |
| YP 003680374.1 | glycoside hydrolase family 48 [Nocardiopsis dassonvillei subsp. dassonvillei DSM 43111] >gb\|ADH67868.1\| glycoside hydrolase family 48 [Nocardiopsis dassonvillei subsp. dassonvillei DSM 43111] glycoside hydrolase family 48 [Cellulomonas flavigena DSM 20109] |
| YP 003638186.1 | >gb\|ADG75987.1\| glycoside hydrolase family 48 [Cellulomonas flavigena DSM 20109] cellulose 1,4-beta-cellobiosidase [Thermobifida fusca YX] |
| YP 290015.1 | >gb\|AAD39947.1\|AF144563-_1 beta-1,4-exocellulase E6 precursor [Thermobifida fusca] >gb\|AAZ55992.1\| cellulose 1,4-beta-cellobiosidase [Thermobifida fusca YX] |
| AAN76734.1 | cellulase Cel48A precursor [Piromyces sp. E2] cellulose 1,4-beta-cellobiosidase [Clostridium thermocellum ATCC 27405] >ref\|ZP_05430126.1\| Cellulose 1,4-beta-cellobiosidase [Clostridium thermocellum |
| YP 001036505.1 | DSM 2360] >ref\|ZP_06247806.1\| Cellulose 1,4-beta-cellobiosidase [Clostridium thermocellum JW20] >gb\|ABN51312.1\| Cellulose 1,4-beta-cellobiosidase [Clostridium thermocellum ATCC 27405] >gb\|EEU01017.1\| Cellulose 1,4-beta-cellobiosidase [Clostridium thermocellum DSM 2360] >gb\|EFB38446.1\| Cellulose 1,4-beta-cellobiosidase [Clostridium thermocellum JW20] |
| CAI06105.1 | 1,4-beta-glucanase [Clostridium thermocellum] glycoside hydrolase family 48 [Ktedonobacter racemifer DSM 44963] |
| ZP 06970946.1 | >gb\|EFH83666.1\| glycoside hydrolase family 48 [Ktedonobacter racemifer DSM 44963] |

### REFERENCES

- Bayer E.A., Y. Shoham, and R. Lamed. 2000. Cellulose-decomposing prokaryotes and their enzyme systems. In M. Dworkin, S. Falkow, E. Rosenberg, K.H. Schleifer and E. Stackebrandt (eds.), The Prokaryotes: An evolving electronic resource for the microbiological community, 3rd edition (latest update release 3.7, September 2001
- Bélaïch. 1997. Characterization of the cellulolytic Complex (cellulosome) produced by Clostridium cellulolyticum. Appl. Env. Microbiol. 63:903-909
- Belaich A, Parsiegla G, Gal L, Villard C, Haser R, Belaich JP.(2002) Cel9M, a new family 9 cellulase of the Clostridium cellulolyticum cellulosome. J Bacteriol., 184(5):1378-84.
- Bélaïch, J.P., C. Tardif, A. Bélaïch, and C. Gaudin. 1997. The cellulolytic system of Clostridium cellulolyticum. J. Biotechnol. 57:3-14
- Chan H. and Doi. R.H. 2000. A large gene cluster for the Clostridium cellulovorans cellulosome. J. Bacteriol. 182:5906-5910
- Doi, R.H., M. Golstein, S. Hashida, J.S. Park, and M. Takagi. 1994. The Clostridium cellulovorans cellulosome. Crit. Rev. Microbiol. 20:87-93
- Dunning, J.W., and E.C. Lathrop. 1945. The saccharification of agricultural residues. Ind. Eng. Chem. 28:350-356; Lee, S.F., C.W. Forsberg, and L.N. Gibbins. 1985. Cellulolytic activity of Clostridium acetobutylicum. Appl. Environ. Microbiol. 50:220-228
- Fierobe HP, Mechaly A, Tardif C, Belaich A, Lamed R, Shoham Y, Belaich JP, Bayer EA. (2001) Design and production of active cellulosome chimeras. Selective incorporation of dockerin-containing enzymes into defined functional complexes. J Biol Chem., 276(24):21257-61
- Fierobe HP, Mingardon F, Mechaly A, Bélaïch A, Rincon MT, Pagès S, Lamed R, Tardif C, Bélaïch JP, Bayer EA.(2005) Action of designer cellulosomes on homogeneous versus complex substrates: controlled incorporation of three distinct enzymes into a defined trifunctional scaffoldin. J Biol Chem. 280(16):16325-34
- Gal, L., S. Pagès, C. Gaudin, A. Bélaïch, C. Reverbel-Leroy, C. Tardif, and J-P. Kakiuchi, M., A. Isui, K. Suzuki, T. Fijino, E. Fujino, T. Kimura, S. Karita, K. Sakka, and K. Ohmiya. 1998. Cloning and DNA sequencing of the genes encoding Clostridium josui scaffolding protein CipA and cellulase CelD and identification of their gene products as major components of the cellulosome. J. Bacteriol. 180:4303-4308
- Gal L, Gaudin C, Belaich A, Pages S, Tardif C, Belaich JP.(1997) CelG from Clostridium cellulolyticum: a multidomain endoglucanase acting efficiently on crystalline cellulose., J Bacteriol. 1997 Nov;179(21):6595-601
- Harris LM, Welker NE, Papoutsakis ET (2002), Northern, morphological and fermentation analysis of spo0A inavtivation and overexpression in C. acetobutylicum ATCC824. J Bacteriol.., 184(13):3586-97
- Heap, JT, Kuehne, SA, Ehsaan, M, Cartman, ST, Cooksley, CM, Scott, JC, and N.P. Minton. 2010. The ClosTron: Mutagenesis in Clostridium refined and streamlined. J Microbiol Methods, 80(1):49-55
- Lamed, R., and E.A. Bayer. 1988. The cellulosome of Clostridium thermocellum. Adv. Appl. Microbiol. 33:1-46
- Lynd, L.R., Van Zyl, W.H., McBride, J.E. and M. Laser,. 2005. Consolidated bioprocessing of cellulosic biomass: an update.Current opinion in Biotechnol. 16:577-583
- Nakhamanovich, B.M., and N.A. Sticheblykina. 1959. Fermentation of pentoses of corn cob hydrolyzates by Clostridium acetobutylicum. Mikrobiologiya 28:91-96
- Nölling J., Breton, G., Omelchenko M.V., Makarova K.S., Zeng Q., Gibson R., Lee H.M., Dubois J., Qui D., Hitti J., GTC Sequencing , Center Production, Finishing, and Bioinformatics Teams, Wolf Y.I., Tatusov R.L., Sabathe F., Doucette-Stamm L., Soucaille P., Daly M.J., Bennett G.N., Koonin E.V. and D.R. Smith. 2001. Genome sequence and comparative analysis of the solvent producing bacterium Clostridium acetobutylicum. J. Bacteriol. 183:4823-4838.
- Reverbel-Leroy C, Belaich A, Bernadac A, Gaudin C, Belaich JP, Tardif C. (1996). Molecular study and overexpression of the Clostridium cellulolyticum celF cellulase gene in Escherichia coli. Microbiology. 142:1013-23.
- Sabathé F, Bélaïch A, Soucaille P.(2002) Characterization of the cellulolytic complex (cellulosome) of Clostridium acetobutylicum. FEMS Microbiol Lett.;217(1):15-22
- Sabathé, F., A. Bélaïch, and P. Soucaille. 2002. Characterization of the cellulolytic complex (cellulosome) of Clostridium acetobutylicum. FEMS Microbiol. Lett. 217:15-22
- Sonderegger M. and Sauer U., 2003. Evolutionary Engineering of Saccharomyces cerevisiae for Anaerobic Growth on Xylose. Appl. Env. Microbiol. 69: 1990-1998
- Soucaille, P., Figge, R. and C. Croux. 2006. Process for chromosomal integration and DNA sequence replacement in clostridia. PCT/EP2006/066997.
- Tamaru, Y., S. Karita, A. Ibrahim, Compere, A.L., and W.L. Griffith. 1979. Evaluation of substrates for butanol production. Dev. Ind. Microbiol. 20:509-517
- Weber, C., Farwick, A., Benisch, F., Brat, D., Dietz, H., Subtil, T. and E. Boles. 2010. Trends and challenges in the microbial production of lignocellulosic bioalcohol fuels. Appl. Microbiol. Biotechnol. 87: 1303-1315

## Claims

1. A genetically modified *Clostridium acetobutylicum* able to grow on cellulose, wherein it comprises a hybrid gene coding for an active family 48 glycoside hydrolase with a dockerin domain that can bind to the cohesin domains of the *Clostridium acetobutylicum* CipA scaffolding protein.

2. The genetically modified *Clostridium acetobutylicum* of claim 1, wherein the hybrid gene comprises the wild type *cel48A* gene of *Clostridium acetobutylicum* which coding sequence of the catalytic domain is replaced with the coding sequence of a an active family 48 glycoside hydrolase catalytic domain while the dockerin of Ce148A remain unchanged.

3. The genetically modified *Clostridium acetobutylicum* of claim 1 or 2, wherein the wild type *cel48A* gene of *Clostridium acetobutylicum* is replaced with the hybrid gene.

4. The genetically modified *Clostridium acetobutylicum* of claim 3, wherein the partial sequence responsible for the inactive catalytic domain in the wild type *cel48A* gene is replaced with a sequence of an active family 48 glycoside hydrolase catalytic domain.

5. The genetically modified *Clostridium acetobutylicum* of one of claims 1 to 4, wherein the hybrid gene comprises a polynucleotide sequence coding for the active family 48 glycoside hydrolase catalytic domain having the amino acids sequence depicted in SEQ ID NO 1

6. The genetically modified *Clostridium acetobutylicum* of one of claims 1 to 5, wherein the dockerin domain binding to the cohesin domain of the C. *acetobutylicum* CipA scaffolding protein has the amino acids sequence depicted in SEQ ID NO 2

7. The genetically modified *Clostridium acetobutylicum* of one of claims 1 to 6, wherein the hybrid gene codes for the hybrid protein having the amino acid sequence depicted in SEQ ID NO 3.

8. The genetically modified *Clostridium acetobutylicum* of one of claims 1 to 7, wherein it comprises additional modifications for the production of bulk chemicals.

9. The genetically modified *Clostridium acetobutylicum* of CLAIM 8, wherein the bulk chemical is selected among the the group consisting of ethanol, butanol, glycerol, 1,2-propanediol, 1,3-propanediol, acetone, isopropanol, acetic acid and lactic acid, and mixtures thereof.

10. An hybrid protein comprising the amino acid sequence depicted in SEQ ID NO 3.

11. An hybrid gene comprising a nucleotide sequence coding for the hybrid protein of claim 10.

12. A method for the preparation of a genetically modified *Clostridium acetobutylicum* comprising a gene coding for an active cellulase being functional in *Clostridium acetobutylicum,* comprising replacing the wild type gene *cel48A* encoding an inactive Ce148A cellulase with a hybrid gene coding for an active family 48 glycoside hydrolase with a dockerin domain that can bind to the cohesin domains of the *Clostridium acetobutylicum* CipA scaffolding protein.

13. The method of one of claims 12, wherein the active cellulase is the hybrid protein of claim 10.

14. A method for the production of a targeted bulk chemical from cellulose, comprising culturing a genetically modified *Clostridium acetobutylicum* as defined in one of claims 1 to 9 on a culture medium comprising cellulose as main source of carbon, and recovering the targeted bulk chemical from the culture medium.

15. The method of claim 14, wherein the genetically modified *Clostridium acetobutylicum* is grown on a membrane reactor.


SEQUENCE LISTING

<110>  INST NATIONAL DES SCIENCES APPLIQUEES
       INSA TOULOUSE

<120>  CELLULOLYTIC CLOSTRIDIUM ACETOBUTYLICUM

<130>  67835 D27164

<160>  2

<170>  PatentIn version 3.5

<210>  1
<211>  626
<212>  PRT
<213>  Clostridium cellulolyticum

<400>  1

Ala Ser Ser Pro Ala Asn Lys Val Tyr Gln Asp Arg Phe Glu Ser Met
1               5                   10                  15

Tyr Ser Lys Ile Lys Asp Pro Ala Asn Gly Tyr Phe Ser Glu Gln Gly
            20                  25                  30

Ile Pro Tyr His Ser Ile Glu Thr Leu Met Val Glu Ala Pro Asp Tyr
        35                  40                  45

Gly His Val Thr Thr Ser Glu Ala Met Ser Tyr Tyr Met Trp Leu Glu
    50                  55                  60

Ala Met His Gly Arg Phe Ser Gly Asp Phe Thr Gly Phe Asp Lys Ser
65                  70                  75                  80

Trp Ser Val Thr Glu Gln Tyr Leu Ile Pro Thr Glu Lys Asp Gln Pro
                85                  90                  95

Asn Thr Ser Met Ser Arg Tyr Asp Ala Asn Lys Pro Ala Thr Tyr Ala
            100                 105                 110

Pro Glu Phe Gln Asp Pro Ser Lys Tyr Pro Ser Pro Leu Asp Thr Ser
        115                 120                 125

Gln Pro Val Gly Arg Asp Pro Ile Asn Ser Gln Leu Thr Ser Ala Tyr
    130                 135                 140

Gly Thr Ser Met Leu Tyr Gly Met His Trp Ile Leu Asp Val Asp Asn
145                 150                 155                 160

Trp Tyr Gly Phe Gly Ala Arg Ala Asp Gly Thr Ser Lys Pro Ser Tyr
                165                 170                 175

Ile Asn Thr Phe Gln Arg Gly Glu Gln Glu Ser Thr Trp Glu Thr Ile
            180                 185                 190

Pro Gln Pro Cys Trp Asp Glu His Lys Phe Gly Gly Gln Tyr Gly Phe

        195                 200                 205

Leu Asp Leu Phe Thr Lys Asp Thr Gly Thr Pro Ala Lys Gln Phe Lys
    210                 215                 220

Tyr Thr Asn Ala Pro Asp Ala Asp Ala Arg Ala Val Gln Ala Thr Tyr
225                 230                 235                 240

Trp Ala Asp Gln Trp Ala Lys Glu Gln Gly Lys Ser Val Ser Thr Ser
                245                 250                 255

Val Gly Lys Ala Thr Lys Met Gly Asp Tyr Leu Arg Tyr Ser Phe Phe
            260                 265                 270

Asp Lys Tyr Phe Arg Lys Ile Gly Gln Pro Ser Gln Ala Gly Thr Gly
        275                 280                 285

Tyr Asp Ala Ala His Tyr Leu Leu Ser Trp Tyr Tyr Ala Trp Gly Gly
    290                 295                 300

Gly Ile Asp Ser Thr Trp Ser Trp Ile Ile Gly Ser Ser His Asn His
305                 310                 315                 320

Phe Gly Tyr Gln Asn Pro Phe Ala Ala Trp Val Leu Ser Thr Asp Ala
                325                 330                 335

Asn Phe Lys Pro Lys Ser Ser Asn Gly Ala Ser Asp Trp Ala Lys Ser
            340                 345                 350

Leu Asp Arg Gln Leu Glu Phe Tyr Gln Trp Leu Gln Ser Ala Glu Gly
        355                 360                 365

Ala Ile Ala Gly Gly Ala Thr Asn Ser Trp Asn Gly Arg Tyr Glu Ala
    370                 375                 380

Val Pro Ser Gly Thr Ser Thr Phe Tyr Gly Met Gly Tyr Val Glu Asn
385                 390                 395                 400

Pro Val Tyr Ala Asp Pro Gly Ser Asn Thr Trp Phe Gly Met Gln Val
                405                 410                 415

Trp Ser Met Gln Arg Val Ala Glu Leu Tyr Tyr Lys Thr Gly Asp Ala
            420                 425                 430

Arg Ala Lys Lys Leu Leu Asp Lys Trp Ala Lys Trp Ile Asn Gly Glu
        435                 440                 445

Ile Lys Phe Asn Ala Asp Gly Thr Phe Gln Ile Pro Ser Thr Ile Asp
    450                 455                 460

Trp Glu Gly Gln Pro Asp Thr Trp Asn Pro Thr Gln Gly Tyr Thr Gly
465                 470                 475                 480

Asn Ala Asn Leu His Val Lys Val Val Asn Tyr Gly Thr Asp Leu Gly
                485                 490                 495

Cys Ala Ser Ser Leu Ala Asn Thr Leu Thr Tyr Tyr Ala Ala Lys Ser
            500                 505                 510

Gly Asp Glu Thr Ser Arg Gln Asn Ala Gln Lys Leu Leu Asp Ala Met
        515                 520                 525

Trp Asn Asn Tyr Ser Asp Ser Lys Gly Ile Ser Thr Val Glu Gln Arg
    530                 535                 540

Gly Asp Tyr His Arg Phe Leu Asp Gln Glu Val Phe Val Pro Ala Gly
545                 550                 555                 560

Trp Thr Gly Lys Met Pro Asn Gly Asp Val Ile Lys Ser Gly Val Lys
                565                 570                 575

Phe Ile Asp Ile Arg Ser Lys Tyr Lys Gln Asp Pro Glu Trp Gln Thr
            580                 585                 590

Met Val Ala Ala Leu Gln Ala Gly Gln Val Pro Thr Gln Arg Leu His
        595                 600                 605

Arg Phe Trp Ala Gln Ser Glu Phe Ala Val Ala Asn Gly Val Tyr Ala
    610                 615                 620

Ile Leu
625

<210>  2
<211>  64
<212>  PRT
<213>  Clostridium acetobutylicum

<400>  2

Phe Ala Asn Thr Ala Thr Pro Gly Asp Val Asn Gly Asp Gly Val Val
1               5                   10                  15

Asn Gly Arg Asp Leu Met Glu Leu Arg Gln Tyr Leu Ala Gly Lys Leu
            20                  25                  30

Asp Ala Ser Lys Ile Asn Leu Ala Ala Ala Asp Val Asn Asn Asp Gly
        35                  40                  45

Val Val Asn Gly Arg Asp Ile Met Glu Leu Thr Lys Leu Ile Ala Lys
    50                  55                  60